# EUROPEAN PATENT APPLICATION

(11) **EP 1 163 926 A1**
(43) Date of publication of application: **19.12.2001**
(21) Application number: 00903965.2
(22) Date of filing: 16.02.2000
(51) Int. Cl.: A61N 1/30

(54) **DEVICE FOR IONTOPHORESIS**

(30) Priority: 24.02.1999 JP 4569699
(71) Applicant: HISAMITSU PHARMACEUTICAL CO. INC., Tosu-shi Saga 841-0017 (JP)
(72) Inventor: INOUE, Kazutaka, Tsukuba-shi, Ibaraki 305-0856 (JP); ADACHI, Hirotoshi, Tsukuba-shi, Ibaraki 305-0856 (JP); MAEDA, Hiroyuki, Tsukuba-shi, Ibaraki 305-0856 (JP); HIGO, Naruhito, Tsukuba-shi, Ibaraki 305-0856 (JP)
(74) Representative: Westendorp, Michael Oliver, Dr.
(86) International application number: JP0000850
(87) International publication number: WO0050119

(57) **Abstract**

This is an iontophoresis device being able to determine a conduction state with high accuracy. A voltage comparator (10B) compares an output current signal (18) indicative of a reactive current flowing through a skin or a mucous with a threshold level (SL) pre-adjusted to a voltage value corresponding to a lower limit of the reactive current, thereby detecting the presence or absence of the reactive current. If the output current signal is higher than the threshold level, the voltage comparator generates an output signal (11B) of "H" to indicate to the controlling circuit that the conduction state is normal. If the output current signal is lower than the threshold level, the voltage comparator generates an output signal (11B) of "L" to indicate to the controlling circuit that the conduction state is abnormal.

## Description

### TECHNICAL FIELD

The present invention relates to a device for use in an iontophoresis apparatus that is applied to transdermal or transmcosal.

### BACKGROUND ART

Iontophoresis is a percutaneous absorption promoting system using electricity, which is based on the principle that forces act on charged molecules such that positively charged molecules transfer from a positive electrode to a negative electrode and negatively charged molecules migrate from the negative electrode to the positive electrode in an electric field generated by passing of electric current, thereby accelerating drug delivery through skin barrier. (Refer to "Journal of Controlled Release", Vol. 18, 1992, pp. 213-220; "Advanced Drug Delivery Review", Vol. 9, 1992, p. 119; and "Pharmaceutical Research", Vol. 3, 1986, pp. 318-326.)

There are conventional means for checking whether the transfer of molecules (including drug) is normally carried out. For example, an iontophoresis apparatus from Motion Control, Inc. determines a value of an output current to that of an output voltage when a direct current is applied to a subject. If the value of the output current is less than a certain value, then it is determined that the transfer of molecules is abnormal, and the application of output voltage is interrupted. However, in this method, a load of a human body at the initial stage of energization may be as high as several MΩ to several tens MΩ at low output voltages and it is difficult to determine whether there is conduction or non-conduction of current. Therefore, a relatively high voltage is applied initially to determine whether there is conduction or non-conduction.

International Publication No. WO96/17651 discloses an apparatus in which the transdermal is first hydrated for a certain length of time before electrically energizing a human body, then the output current is measured and the output voltage is interrupted if the measured output current is out of a certain range. International Publication No. WO88/08729 discloses another related technique, i.e., an apparatus in which when an over-current flows, the supply of output current is terminated.

With these apparatus, there are problems about the DC impedance used as a measure means of conduction such that differences in individual are large and that the impedance is high and even higher at lower output voltages and that the impedance is highly sensitive to the hydration conditions. Therefore, conventionally, conduction in iontophoresis cannot be determined with a sufficient level of accuracy.

In order to overcome the aforementioned problems, although it is considered in such a way that a relatively high voltage at the initial stage of energization may be applied or sufficient hydration of the transdermal may be employed to increase detection sensitivity of impedance, it produces new problems that users may feel uncomfortable or that the states of current conduction cannot be measured until the hydration is sufficient.

An object of the present invention is to overcome the aforementioned problems and to provide a device for iontophoresis that can determine conduction states with a high level of accuracy.

### DISCLOSURE OF THE INVENTION

The inventors have concentrated in order to achieve the aforementioned object. The inventors focussed on the capacitance that exists in the transdermal or the transmcosal, and they found that detecting the current (reactive current) that flows through the capacitance or the charges (residual voltage) stored in the capacitance allows determining of the conduction states, and then made the present invention accordingly. The present invention allows determining of not only conduction states due to complete detachment of a part for applying iontophoresis to the transdermal or poor contact at a terminal or cracks in the electrode, but also the conditions of the transdermal to which iontohoresis is applied.

A device for iontophoresis according to the present invention comprises first means for detecting a capacitance stored in transdermal or transmcosal and second means for determining a conduction state of current into the transdermal or the transmcosal based on the output detected by the first means. The first means can be, for example, a detection circuit for a reactive current flowing through the transdermal or the transmcosal or a detection circuit for a residual voltage developed in the transdermal or the transmcosal. If the detection circuit of the reactive current is used, the voltage applied to the subject has a waveform such as an AC wave, a rectangular wave, or a DC wave on which a rectangular wave is superimposed, or a DC wave on which an AC wave is superimposed. If the detection circuit of residual voltage is used, the voltage applied is an intermittent waveform.

With a method for determining the operation of an iontophoresis apparatus according to the present invention, a capacitance in the transdermal or the transmcosal is detected to determine the operation of a conduction state of current through the transdermal or the transmcosal. The capacitance is detected by detetecting a reactive current that flows through the transdermal or the transmcosal, or detecting a residual voltage developed in the transdermal or the transmcosal.

An iontophoresis apparatus according to the present invention comprises a preparation for iontphoresis, holding a drug, and a device for iontophoresis having means for generating an electrical output to supply a drug from the preparation into the transdermal or the transmcosal and means for detecting a value indicative of a capacitance of the transdermal or the transmcosal to determine whether conduction of electrical output into the transdermal or transmcosal is normal or abnormal.

The aforementioned configuration makes it possible to accurately determine the conduction state for an iontophoresis apparatus.

### BRIEF DESCRIPTION OF THE DRAWINGS

FIG. 1 is a general cross-sectional view illustrating an example of an iontophoresis apparatus according to the present invention;
FIG. 2 illustrates an example of an output current detecting circuit;
FIG. 3 illustrates an example of an output current detecting circuit;
FIGS. 4(a)-(d) illustrate voltage waveforms or current waveforms on various parts;
FIG. 5 illustrates an example of a residual voltage detecting circuit;
FIG. 6 illustrates a voltage waveform across the output terminals of the residual voltage detecting circuit;
FIG. 7 illustrates a device for iontophoresis that incorporates a reactive current detecting circuit; and
FIG. 8 illustrates a device for iontophoresis that incorporates a residual voltage detecting circuit.

### BEST MODE FOR CARRYING OUT THE INVENTION

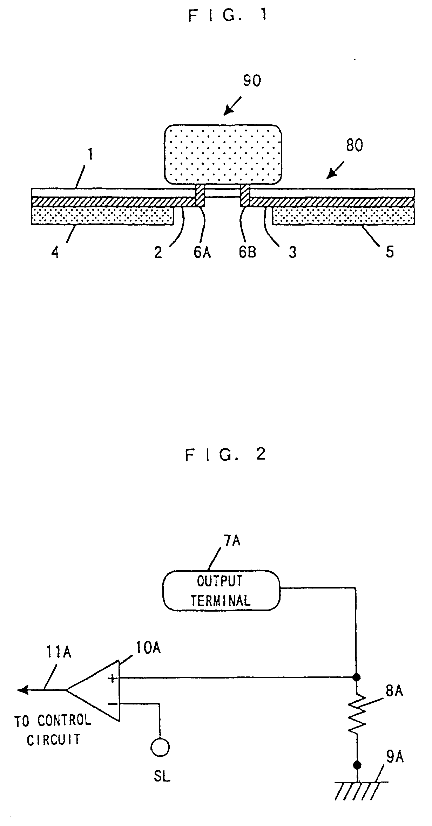

FIG. 1 is a general cross-sectional view illustrating an example of an iontophoresis apparatus according to the present invention. As shown in the figure, the apparatus comprises a preparation 80 for iontophoresis, which holds a drug or drugs and a device 90 for iontophoresis, which serves as a power supply for generating an electrical output that supplies the drug from the preparation to the transdermal or the transmcosal. The preparation 80 comprises an insulation substrate 1, a drug reservoir side electrode 2, an electrolyte reservoir side electrode 3, a drug reservoir 4, an electrolyte reservoir 5, and tabs 6A and 6B for detachably attaching a device 90 to the preparation 80. The tabs 6A and 6B are connected to the electrodes 2 and 3, respectively. The device 90, as mentioned below, includes a circuit that detects a value reflecting a capacitance of the transdermal or the transmcosal to determine whether the electrical output flows normally through the transdermal or the transmcosal.

An iontophoresis apparatus according to the present invention can be of any type that can be used in a conventional manner. In other words, the apparatus comprises a power supply, electrodes, at least one drug reservoir, and at least one electrolyte reservoir. (If there are two or more drug reservoirs, the electrolyte-holding reservoir is not essential). The drug reservoir and electrolyte reservoir may be attached to, for example, the skin or the transmcosal directly or indirectly.

When the iontophoresis apparatus is operated, charges are stored in the transdermal or the transmcosal. This is referred to as a capacitance of the transdermal or the transmcosal. For example, when the drug reservoir and electrolyte reservoir are not in intimate contact with the transdermal etc., the capacitance of the transdermal etc. causes a decrease in reactive current and a decrease in charge (an element determining a time constant of the residual voltage) stored in the skin. When this happens, for an output voltage, it causes that the reactive current does not reach a predetermined value or the residual voltage does not reach a predetermined value. Thus, detecting the the reactive current or residual voltage allows accurate, quick determining of current a conduction state of the iontophoresis apparatus, thereby being able to prevent an abnormal condition.

When abnormal conditions occur such as damage to the skin, short-circuit due to poor printing of conductive paste during the manufacture of the drug reservoir side electrode and the electrolyte reservoir side electrode, or short-circuit etc. due to perspiration etc., the DC impedance prominently drops so that the DC current exceeds a predetermined value. Thus, these abnormal conditions can be determined by detecting a DC impedance as in the conventional art.

The impedance of the capacitance (described below as capacity) of the transdermal and the transmcosal. for example, described in "IYODENSHI TO SEITAIKOGAKU" Vol. 11, No. 5, pp337-343. According to this document, using an electrode about 9 mm (0.64 cm²), subjecting the transdermal to hydration for more than 30 minutes, and supplying a current for a predetermined length of time, then, the result of the measurement is that the impedance of the transdermal is about 1.8 kΩ on an AC signal of 10 kHz (calculated as Rp: 4 kΩ and Cp: 0.008 µF) and is about 0.36 kΩ on an AC signal of 100 kHz (calculated by assuming that Rp = 0.4 kΩ and Cp = 0.002µF). Rp, here, is a resistance component of an equivalent circuit of the skin impedance and Cp is a capacity component. Detection of the reactive current is to measure a current that flows through the impedance of the capacity for AC current.

While the time constant of a living organism for DC current is about 6 ms (calculated as Rp: 100 kΩ and Cp: 0.06 µF), when no load is connected to the apparatus, there is no capacity and therefore a time constant seen from the apparatus is uncertain. Thus, a discharging resistor of, for example, 100 kΩ to 1MΩ is used in the circuit to provide a time constant of 0 ms. Detection of residual voltage is to measure the difference in time constant between them.

When the device for iontophoresis uses a waveform such as an AC waveform, a rectangular waveform, a DC waveform to which a rectangular waveform is superimposed, or a DC waveform to which an AC waveform is superimposed, a current is stored into the capacity of the skin during the positive period of the time-varying component and discharged from the capacity during the negative period. A reactive current that flows through the skin is detected by thus repeatedly storing and discharging the current.

When an intermittent current is used, a charge (voltage) is stored in the capacity of the skin by conducting the output, and the charge remains in the capacity during the off-period of the output and causes a voltage so that a residual voltage is measured.

In order to know the conduction states for the iontophoresis apparatus, it is sufficient to measure either the reactive current or the residual voltage.

Moreover, a display function such as a light emitting diode (LED) or a buzzer and a function for adjusting or interrupting the output may be added to the device for iontophoresis according to the present invention, in order to indicate to the user when an abnormal condition is determined by detecting the reactive current or residual voltage.

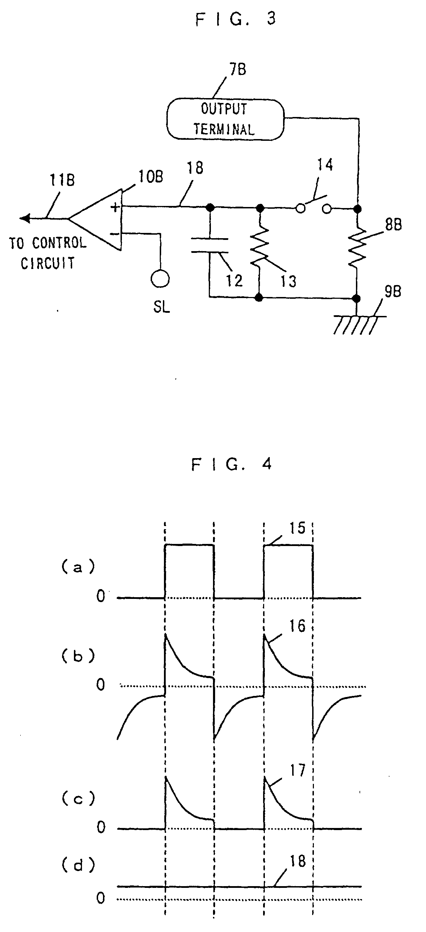

FIG. 3 illustrates an example of a detecting circuit for output current to detect a reactive current resulting from the capacity of the skin. In this embodiment, the output waveform superimposed with frequency components is used. Referring to FIG. 3, reference numerals 7B and 8B denote a negative output terminal and a current-detecting fixed resistor, respectively. Reference numerals 9B, 10B, and 11B denote a circuit ground, a voltage comparator, and an output signal from the voltage comparator, respectively. Reference numerals 12, 13, and 14 denote a current-storing capacitor, a discharging fixed resistor, and an analog switch, respectively.

FIGS. 4(a)-(d) illustrate voltage waveforms or current waveforms, respectively. When the output is a rectangular waveform 15 having a frequency of 10 kHz and a duty cycle of 50% as shown in FIG. 4(a), the current outputted from the negative output terminal 7B is converted into an output current waveform 16 as shown in FIG. 4(b) by the fixed resistor 8B. By an analog switch 14 in synchronism with the output, the output current waveform 16 is passed as only the positive current waveform 17 as shown in FIG. 4(c) to a capacitor 12 of the succeeding stage, and then an output current signal 18 as shown in FIG. 4(d) is produced by smoothing out the waveform.

The voltage comparator 10B compares the output current signal 18 indicative of the reactive current that flows through the skin or the mucous with a threshold level SL pre-adjusted to avoltage value corresponding to a lower limit of the reactive current, thereby determining whether a reactive current exists. When the output current signal 18 is higher than the threshold level SL, the voltage comparator 10B outputs an output signal 11B of "H" to indicate to a control circuit that a conduction state is normal. When the output current signal 18 is lower than the threshold level SL, the voltage comparator 10B outputs the output signal 11B of "L" to indicate to the control circuit that a conduction state is abnormal.

The threshold level can be set to a voltage value that corresponds to a reactive current in the range of 0.01 to 10 mA and more preferably 0.1 to 1 mA, so that the conduction state may be detected even at low voltages. In order to detect poor intimate contact, a D/A converter can be used to adjust the threshold level in accordance with the area of the preparation applied to the subject and the output voltage. Also, an A/D converter is used in place of the voltage comparator 10B to change detection conditions in accordance with the output voltage etc. Additionally, a voltage amplifier for amplifying the output signal 18 may be added to the forward stage etc. of the voltage comparator 10B, or a feedback signal of the output voltage may be used as the threshold level, so that the threshold level has a voltage value proportional to the output voltage.

For the detection of a reactive current, a sample-and-hold circuit may conveniently be used to detect positive portions of the reactive current. Instead of detecting only the reactive current, the current including a current for conveying the drug may be detected, or a part of the reactive current may be detected. Further, a function for detecting a part of the DC current may be provided.

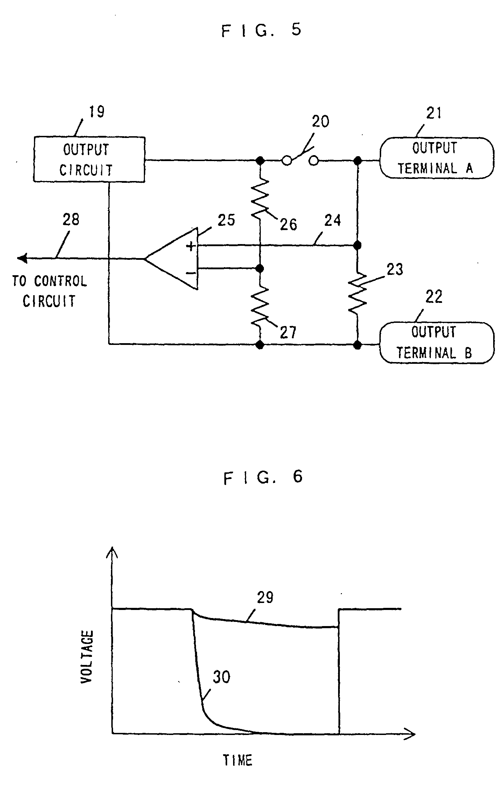

FIG. 5 illustrates an example of a detecting circuit for residual voltage to detect the residual voltage developed across the skin (load). In the present embodiment, an intermittent energization is used. Referring to FIG. 5, reference numerals 19, 20, and 21 denote an output circuit, an analog switch, and an output terminal A, respectively. Reference numerals 22, 23, and 24 denote an output terminal B, a discharging fixed resistor, an output signal to the voltage comparator, respectively. Reference numerals 25, 26 and 27, and 28 denote a voltage comaprator, voltage adjusting fixed resistors, and an output signal from the voltage comparator, respectively.

FIG. 6 illustrates voltage waveforms across the output terminals. Referring to FIG. 6, a voltage waveform 29 describes the output signal across the output terminals when a load is connected across the output terminals and a voltage waveform 30 represents the output signal across the output terminals when no load is connected across the output terminals.

The DC output voltage from the output circuit 19 is usually outputted to the output terminal 21 through the analog switch 20. When the residual voltage is detected, the analog switch 20 is a non-conduction state by a signal issued from the control circuit. Then, the voltage comparator 25 compares the output signal 24 when the output voltage sent to the output terminal 21 is interrupted, with the threshold level that is produced by dividing the output voltage from the output circuit 19 by the fixed resistors 26 and 27. If the output signal 24 indicative of the residual voltage stored in the skin or the mucous is higher than the threshold level, the voltage comparator 25 generates an output signal 28 of "H" , indicating to the control circuit that the conduction state is normal. Conversely, if the output signal 24 is lower than the threshold level, the voltage comparator 25 generates the output signal 28 of "L" , indicating to the control circuit that the conduction state is abnormal.

The fixed resistor 23 is provided to prevent the output terminal 21 from being open-circuited when the analog switch 20 is the non-conduction state and there is no loading, and to allow the residual voltage on the output terminal 21 to discharge with an arbitrary time constant when the analog switch 20 is the non-conduction state and there is a loading. The output signal 24 may be obtained by dividing a voltage on the output terminal 21 or a signal with a level lowered to a voltage level for the control circuit, and then which may be provided in a variety of ways. Likewise, the threshold level may be modified in a variety of ways just as in the detection of the reactive current.

The residual voltage is measured or detected in synchronism with the interruption of the output voltage and therefore the measurement or detection of the residual voltage can be performed in several microseconds to several seconds without affecting the output. Just as in the measurement of the reactive current, adjusting the time constant and digitizing the voltage with an A/D converter, or adjusting the time between the interruption and reading-in allows determining of whether the preparation is in intimate contact with the transdermal or the transmcosal.

The output waveform used for detecting the reactive current in the present invention contains a DC component for drug dosage and a frequency component for detection of a capacitive impedance. Means for detecting the impedance may be one that integrates or peak-holds the current resulting from repetitive application of pulses, or one that performs the detection in real time with one shot of pulse triggered. If detection of impedance at all times is not required, then a frequency component may be superimposed to the output signal at an arbitrary timing to detect the impedance of the capacity. The waveform of an intermittent output signal used in detecting the residual voltage may be one in which a DC current is interrupted at an arbitrary time intervals and the residual voltage remaining on the load is measured immediately after the output is interrupted. Thus, observing the voltage remaining on the load allows detection of impedance of the capacity.

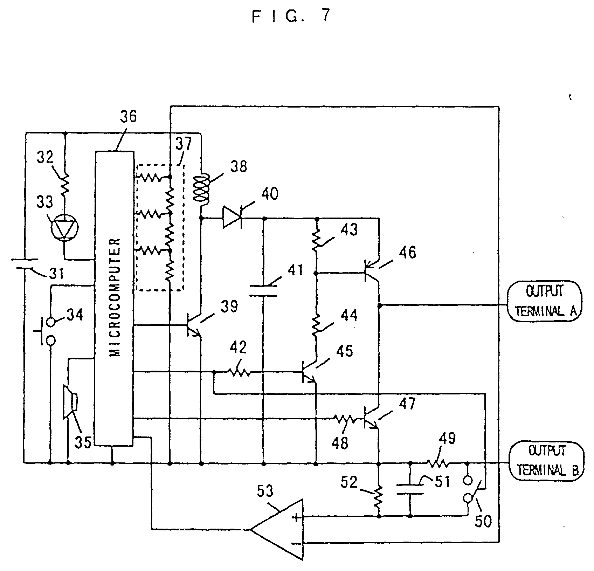

FIG. 7 illustrates a device for iontophoresis having a circuit for detecting the reactive current shown in FIG. 3. Referring to FIG. 7, reference numerals 31 and 32 denote a battery and a fixed resistor that limits the current flowing through a light-emitting diode (LED), respectively. Reference numerals 33, 34, and 35 denote an LED, a power switch, and a buzzer, respectively. Reference numerals 36, 37, and 38 denote microcomputer, a D/A converter, and a boosting coil, respectively. Reference numerals 39, 40, and 41 denote a coil-driving transistor, a rectifying diode, and rectifying capacitor, respectively. Reference numerals 42, 43, 44, and 48 denote fixed resistors that limit current. Reference numerals 45, 46, 47 denote output transistors. Reference numerals 49, 50, 51, 52, and 53 denote a current detecting fixed resistor, an analog switch, a current storing capacitor, a discharge fixed resistor, and a voltage comparator, respectively.

The essential operation of the device according to the invention will now be described with reference to the figures. When the power switch 34 is pressed, the microcomputer 36 is activated to start to give a drug to a subject under a specific procedure previously programmed. The microcomputer 3 6 causes the LED 33 to light up and then causes the transistor 39 to oscillate to boost the voltage of the battery 31. When the transistor 39 oscillates, a back electromotive force is developed across the coil 38 and charges the capacitor 41 through the diode 40. The microcomputer 36 controls the transistors 45, 46 and 47, which are repeatedly conducted and non-conducted in opposite phase, and therefore the voltage across the capacitor 41 is outputted in the form of a rectangular wave having a certain frequency to the output terminal A. If a load has been connected across the output terminals A and B, a current in accordance with the impedance of the load flows through the output terminal B, causing a current waveform to appear across the fixed resistor 49 in accordance with the amount of current. The analog switch 50 operates to sample-and-hold only the positive waveform of the current. The sampled positive waveform is smoothed out by the capacitor 51 and is then outputted to the positive input of the voltage comparator 53. The negative input of the voltage comparator 53 receives the threshold level corresponding to a lower limit of the reactive current, which is the output signal of the microcomputer 36 having been digital-to-analog converted by the D/A converter 37.

The voltage comparator 53 compares these two inputs and provides the comparison result to the microcomputer 36. If the output signal of the voltage comparator 53 is "H", the microcomputer 36 determines that conduction is normal. If the output signal of the voltage comparator 53 is "L", the microcomputer 36 determines that conduction is abnormal. When the conduction is abnormal, the LED 33 flashes and the buzzer 35 sounds to warn the operator. If the abnormal conduction state is not remedied a certain length of time after the warning, the output is interrupted and the operator is informed that the output has been interrupted, the buzzer 35 continuing to sound and the LED 33 being de-energized. Performing these operations in sequence secures the safety of the user.

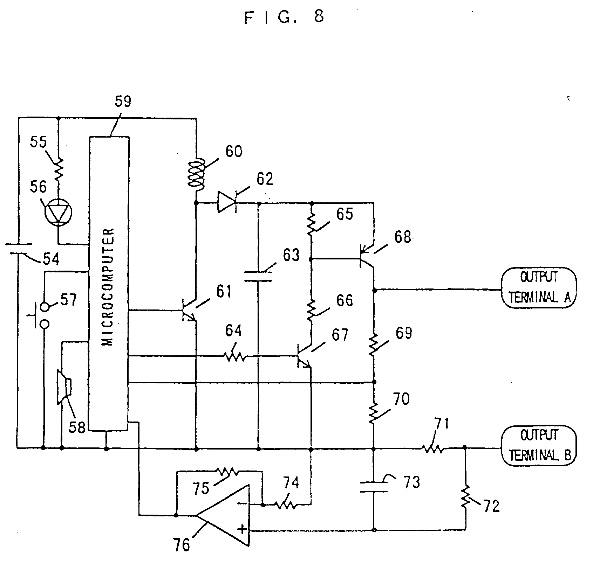

FIG. 8 illustrates an iontophoresis device having a circuit for detecting a residual voltage, using amicrocomputer. Referring to FIG. 8, reference numerals 54, 55, and 56 denote a battery, a fixed resistor that limits the current flowing through an LED, and an LED, respectively. Reference numerals 57, 58, and 59 denote a power switch, a buzzer, and a microcomputer that incorporates an A/D converter, respectively. Reference numerals 60, 61, and 62 denote a boosting coil, a transistor for driving the coil, and a rectifying diode, respectively. Reference numerals 63, 64-66, and 67-68 denote a rectifying capacitor, fixed resistors that limit current, and output transistors, respectively. Reference numerals 69-70, 71, and 72 denote discharging fixed resistors for detecting the residual voltage, fixed resistors for detecting the current, a fixed resistor for limiting current, respectively. Reference numerals 73, 74-75, and 76 denote a current-storing capacitor, a fixed resistor for adjusting an amplification factor, and a voltage amplifier, respectively.

The basic operations of the device according to the present invention will now be described with reference to the figures. When the power switch 57 is pressed, the microcomputer 59 is activated to start to give a drug to a subject under a procedure previously programmed. The microcomputer 59 causes the LED 56 to light up and then causes the transistor 61 to oscillate, thereby producing a voltage that is boosted from the voltage of the battery 54. When the transistor 61 oscillates, a back electromotive force is developed across the coil 60, the back electromotive force being stored in the capacitor 63 through the diode 62. When the microcomputer 59 controls the transistor 67 to conduct the transistor 68, the voltage held across the capacitor 63 is directed to the output terminal A.

When the transistor 68 is then turned off, a residual voltage appears on the output terminal A if a load has been connected to the output terminals. The residual voltage is divided by fixed resistors 69 and 70 and is then directed to the analog input terminal of the A/D converter in the microcomputer 59. When the measured voltage has reached a predetermined value, the microcomputer 59 determines that the conduction state is normal. When the measured voltage has not reached the predetermined value, the microcomputer 59 determines that the conduction state is abnormal. If the conduction state is abnormal, then the microcomputer 59 causes the LED 56 to flash and the buzzer 58 to sound, thereby warning the operator. If the abnormal condition is not solved beyond a certain period of time, then the microcomputer 59 shuts off the output. Then, the microcomputer 59 causes the buzzer to sound and the LED to go off, thereby indicating to the user that the output has been interrupted. Performing these operations in sequence secures the safety of the user.

If a constant current means, which comprises circuit elements 71-76 to keep the current (current for giving a drug) flowing through the DC impedance to a predetermined value, is used as the output controlling means, an excess current is to be prevented from flowing through the load so that safety is improved. When the preparation comes off or floats from the applied part of the human body, a general iontophoresis apparatus may not only fail to give a sufficient amount of drug to the applied part but also cause concentration of current in areas still in intimate contact with it. This may result in excessive supply of drug in particular small areas and electric burn. In contrast to this, the iontophoresis apparatus according to the present invention electrically energizes the body while making sure that a reliable electrical path is established for giving a drug. If an abnormal impedance should be detected, the apparatus warns the user, requesting of an improvement or interrupting the output, if necessary, to ensure safety of the user.

### Examples

### (Example 1)

A device for iontophoresis incorporates a detecting circuit for an output current as shown in FIG. 3, which used a rectangular wave having a frequency of 10 kHz and a duty cycle of 50% as an output voltage that was variable from 0 V to 10 V in increments of 2 V. The detection conditions were as follows. The current-detecting fixed resistor 8B was 1 kΩ. The current-storing capacitor 12 was 0.1 µF. The discharging fixed resistor 13 was 1 MΩ. The threshold level of the voltage comparator 10B was adjusted to 0.1 V. When the iontophoresis apparatus incorporating the aforementioned iontophoresis device was used, each of the positive and negative electrodes applied to the skin had an area of 5 cm².

### (Example 2)

A structure of the device for iontophoresis of Example 2 is the same as that of Example 1, which used a rectangular wave having a frequency of 10 kHz and a duty cycle of 50 % as the output voltage that was adjusted to a 5 V. The detection conditions were as follows. The current-detecting fixed resistor 8B was 1 kΩ. The current-storing capacitor 12 was 0.1 µF. The discharge fixed resistor 13 was 1 MΩ. The threshold level of the voltage comparator 10B was adjusted to 0.5 V, which was 1/10 of the output voltage. When the iontophoresis apparatus incorporating the aforementioned iontophoresis device was used, each of the positive and negative electrodes applied to the skin had an area of 5 cm².

### (Example 3)

A device for iontophoresis incorporates a detecting circuit for a residual voltage as shown in FIG. 5, in which the output voltage of the output circuit 19 was adjusted to a DC voltage of 5 V, and the analog switch 20 was closed to direct 5 V to the output terminal 21 and then the analog switch 20 was opened. The detection conditions were as follows . The voltage-adjusting fixed resistor 26 was 10 kΩ. The voltage-adjusting fixed resistor 27 was 90 kΩ. The threshold level of the voltage comparator 25 was 4.5 V, which was 9/10 of the output voltage. When the iontophoresis apparatus incorporating the aforementioned iontophoresis device was used, each of the positive and negative electrodes applied to skin had an area of 5 cm².

### (Comparison 1)

A device for iontophoresis incorporates a detecting circuit for an output current as shown in FIG. 2. The detecting circuit for the output current comprises a negative-electrode side output terminal 7A, a current-detecting fixed resistor 8A, a circuit ground 9A, and a voltage comparator 10A as shown in FIG. 2, obtaining an output signal 11A from the voltage comparator 10A. In this embodiment, a DC voltage was used as the output voltage that was variable from 0 V to 10 V in increments of 2V. The detection conditions were as follows. The current-detecting fixed resistor 8A was 1 kΩ and the threshold level of the voltage comparator 10A was 0.1 V. When the iontophoresis apparatus incorporating the aforementioned iontophoresis device was used, each of the positive and negative electrodes applied to the skin had an area of 5 cm².

### (Test 1)

For Example 1 and Comparison 1, Table 1 lists the input voltages to the voltage comparators 10A and 10B and the output signals 11A and 11B with different values of the output voltage.

**Table 1**

| Output voltage (V) | Example 1 | | Comparison 1 | |
|---|---|---|---|---|
| | Input voltage (V) | Detection of conduction | Input voltage (V) | Detection of conduction |
| 0 | 0 | × | 0 | × |
| 2 | 0.19 | ○ | 0 | × |
| 4 | 0.43 | ○ | 0 | × |
| 6 | 0.69 | ○ | 0 | × |
| 8 | 0.97 | ○ | 0.03 | × |
| 10 | 1.24 | ○ | 0.12 | ○ |

If the output signal (detection of conduction) is "H" (indicated by ○), then it is determined that conduction is normal. If the output signal is "L" (indicated by X), then it is determined that conduction is abnormal. As shown in Table 1, in Example 1, conduction was observed at an output voltage equal to or higher than 2 V. In contrast to this, in Comparison 1, conduction was observed only at an output voltage of 10 V. This indicates that the circuit according to Example 1 has higher detection accuracy than Comparison 1.

### (Test 2)

In Example 2, a detachment test was conducted with 3 subjects to determine whether the device was detached from the applied part of the transdermal. The result is as follows.

**Table 2**

| Area of the applied part | Subject 1 | | Subject 2 | | Subject 3 | |
|---|---|---|---|---|---|---|
| | Voltage (V) | Detection of detachment | Voltage (V) | Detection of detachment | Voltage (V) | Detection of detachment |
| 0/10 | 0 | L | 0 | L | 0 | L |
| 2/10 | 0.17 | L | 0.17 | L | 0.18 | L |
| 4/10 | 0.29 | L | 0.30 | L | 0.31 | L |
| 6/10 | 0.39 | L | 0.40 | L | 0.41 | L |
| 8/10 | 0.47 | L | 0.48 | L | 0.48 | L |
| 10/10 | 0.53 | H | 0.55 | H | 0.56 | H |

The test was conducted in such away that the output voltage was 5 V, and the device was peeled off the transdermal slowly so that the area of the device in contact with the applied part of the transdermal varies from 0/10 to 10/10. For different sizes of the applied area, the input voltage and output signal 11B (detection of detachment) of the voltage comparator 10B were measured. "L" of the output signal 11B indicates that conduction was abnormal and "H" indicates that conduction was normal. From Table 2, the iontophoresis device according to Example 2 can detect detachment of the device encountered, even a small area, during the application.

### (Example 3)

In Example 3, a detachment test was conducted in the same manner as Example 2. The result is as follows.

**Table 3**

| Area of the applied part | Subject 1 | | Subject 2 | | Subject 3 | |
|---|---|---|---|---|---|---|
| | Voltage (V) | Detection of detachment | Voltage (V) | Detection of detachment | Voltage (V) | Detection of detachment |
| 0/10 | 0 | L | 0 | L | 0 | L |
| 2/10 | 1.6 | L | 1.6 | L | 1.5 | L |
| 4/10 | 2.7 | L | 2.6 | L | 2.6 | L |
| 6/10 | 3.7 | L | 3.6 | L | 3.5 | L |
| 8/10 | 4.2 | L | 4.2 | L | 4.1 | L |
| 10/10 | 4.8 | H | 4.8 | H | 4.7 | H |

The test was conducted in such away that the output voltage was 5 V, and one of the areas of the device in contact with the applied part of the transdermal was varied from 0/10 to 10/10. The voltage on the output terminal 21 and the output signal 28 were measured 1 ms after the analog switch 20 was opened. "L" of the output signal 28 (detection of detachment) indicates that conduction was abnormal and "H" represents that conduction was normal. From Table 3, the iontophoresis device according to Example 3 can detect detachment of the device encountered, even a small area, during the application.

### INDUSTRIAL APPLICABILITY

The iontophoresis device according to the present invention can detect conduction states with high accuracy and is applicable to iontophoresis in the field of medical care.

## Claims

1. A device for iontophoresis supplying a drug to transdermal or transmcosal, comprising: first means for detecting a capacitance stored in the transdermal or the transmcosal; and second means for determining a conduction state of current into the transdermal or the transmcosal based on the output detected by the first means.

2. The device for iontophoresis according to Claim 1, wherein the first means is a detection circuit for a reactive current flowing through the transdermal or the transmcosal.

3. The device for iontophoresis according to Claim 1, wherein the first means is a detection circuit for a residual voltage developed in the transdermal or the transmcosal.

4. A method for determining an operation of an iontophoresis apparatus, wherein a capacitance stored in transdermal or transmcosal is detected to determine a conduction state of current flowing into the transdermal or the transmcosal.

5. The method for detecting an operation of an iontophoresis apparatus according to Claim 4, wherein the detection of the capacitance is carried out by detecting a reactive current flowing through the transdermal or the transmcosal.

6. The method for detecting an operation of an iontophoresis apparatus according to Claim 4, wherein the detection of the capacitance is carried out by detecting a residual voltage developed in the transdermal or the transmcosal.

7. An iontophoresis apparatus comprising: a preparation for iontophoressis, holding a drug; and a device for iontophoresis having means for generating an electrical output to supply a drug from the preparation into transdermal or transmcosal and means for detecting a capacitance stored in the transdermal or the transmcosal to determine a conduction state of a current flowing into the transdermal or the transmcosal.
